# EUROPEAN PATENT APPLICATION

(11) **EP 2 168 501 A1**
(43) Date of publication of application: **31.03.2010**
(21) Application number: 08775415.6
(22) Date of filing: 30.05.2008
(51) Int. Cl.: A61B 10/06

(54) **MEDICAL DEVICE FOR BIOPSIES**

(30) Priority: 04.06.2007 ES 200701527
(71) Applicant: Soriano Romero, Francisco Santiago, 08027 Barcelona (ES)
(72) Inventor: Soriano Romero, Francisco Santiago, 08027 Barcelona (ES)
(74) Representative: Isern-Jara, Nuria
(86) International application number: PCT/ES2008/000387
(87) International publication number: WO 2008/148903

(57) **Abstract**

Medical device for biopsies equipped with an elongated flexible section designed to be inserted through a conduit of a surgical instrument, said elongated section comprising an interior tube and an exterior tube each capable of moving longitudinally with respect to the other; a grip device located at the distal end of the elongated section and designed for taking a sample; controls situated at the proximal end of the elongated section using which the medical device for directing the grip device is manipulated, said controls being connected to means of suction. The controls include a handle equipped with a closed receptacle with connected thereto the suction means and the proximal end of the interal tube of the elongated section, in such a way that the sample taken is pulled through the internal tube by the suction force to be deposited in said container.

## Description

### Field of the invention

The present invention relates to a medical device for biopsies provided with an elongated flexible section designed to be inserted through a conduit of a surgical instrument, comprising said elongated section an inner tube and an outer tube each capable of moving longitudinally with respect to the other; a gripping means located at the distal end of the elongated section, and designed to be opened at the precise site at which the tissues are extracted and for taking a sample; controls located at the proximal end of the elongated section from which the medical device is manipulated so as to direct the gripping means between an opening and a closing position, said controls being connected to a suction means suitable for generating a suction force.

### Background of the invention

There are currently available on the market surgical devices of the pincer type for endoscopic biopsies of multiple configurations. The commonest form is that which consists of at its distal end two small arms acting as a pincer once they have been introduced into a human or animal body through a tube, so as to be able to take, for example, tissues samples from specific areas and even to be able to remove whole areas of tissue. In addition, devices of this type also allow carrying out certain treatments in difficult access areas of the human body. Due to its versatility, the present invention is not limited to the field of endoscopy, but it embraces all the application fields of those surgical devices with a pincer or scissor function, such as, for example, heart biopsy operations and laparoscopies.

Generally, the surgical devices of the type described comprise a traction cable which is coupled to the scissor or pincer arms through some lever elements. When the traction cable is pushed, said scissor or pincer arms are opened, and when the cable is pulled they close, so that the force applied through the cable traction in order to raise and take a sample is minimal. Subsequently, the sample taken is extracted by removing the surgical device from inside the tube, passing through the entire length of the tube on its way out. Once outside the tube, the pincer is opened and the sample is tanked in a container in order to be analysed.

It is currently recommended to carry out from 6 biopsies per operation, or even between 3 and 4 biopsies per operation. As has been described above, each extraction consists in a manoeuvre that requires great precision in order to avoid damaging tissues and the maintenance of very strict conditions of hygiene so that the sample taken is not contaminated. Moreover, it is important that such a manoeuvre is performed as rapidly as possible, thus avoiding extending unnecessarily the duration of the operation, whereby unfortunately in practice only 1 biopsy is carried out per operation. For this, any improvement made to a medical device of this type is considered to be of great significance.

The European patent Application EP0065054 describes a medical device comprising a hollow cylinder and a cutting means. The cutting means consists of two tongues formed by cuts made in the cylinder itself in the form of strips which extend longitudinally and whose distal ends are folded in a perpendicular way towards the centre of the open base of the cylinder. When the distal ends of the tongues are brought closer together, partially closing the base of the cylinder, the tongues are capable of cutting a tissue interposed between them. The main disadvantage of such a device is that it only allows taking small samples, and in addition the tongues need to be very sharp in order to work properly, which makes their manufacturing considerably more complicated.

In the international Application WO95/08291 a description is given of a medical device comprising an internal tube whose distal end is designed to perforate a tissue so as to take a sample thereof through suction caused by the generation of a depression. With said device it is difficult for the internal tube to sink far enough into the wall of the tissue to be able to take a sample of adequate size, and for the skilled person using it, it is difficult to have to push the internal tube so deeply into the wall of the tissue.

The American patent Application US6110127 describes a medical device to be used in combination with an endoscope. The device comprises a tube designed to be inserted in an endoscope and some controls to direct the device. The tube consists of an inner and an outer tube capable of moving forwards and backwards in a direction that is axial in relation to the inner tube. The controls are arranged at the distal end of the tube, which is in its turn connected to a suction means which generates a force of suction. A means of taking samples is arranged at the distal end of the tube and are designed so that a portion of tissue can be cut and subsequently sucked up by the inner tube and tanked inside the medical device. In a first embodiment mode of described in the application US6110127, the samples which are taken with the medical device are accumulated at the end of the inner tube in an area specially designed for this purpose. In a second embodiment mode, the proximal end of the tube is connected not only to a suction means, but also to a tank of water, so that each sample taken is cleaned with the water and the air found in the inner tube, and subsequently sucked along the entire length of the tube to the opposite end, which gives onto a container equipped with a filter and with a tank to contain the water thus sucked in. The main disadvantage of this embodiment is its complexity, which makes its manufacturing more expensive and increases its chances of developing a malfunction. Moreover said medical device needs to be properly cleaned after each extraction before being used again, which makes its use even more inconvenient.

### Summary of the invention

The medical device for biopsies object of the present invention eliminates all the disadvantages mentioned in the state of art and essentially it is **characterized in that** controls include a handle provided with a closed container to which is connected a suction means and the proximal end of the inner tube of the elongated section, in such a way that the sample taken is pulled through the inner tube by the force of suction generated until it is deposited in the container. In this way it is possible to avoid the need to extract and re-introduce completely the medical device through the canal of a surgical instrument more than once.

The medical device object of the present invention is also characterized by its gripping means which is equipped with a cannula located at the distal end of the inner tube, through which the sample is sucked by the force of the suction generated. Thanks to said cannula, it is possible to direct the end of the medical device and position it in a precise and easy way.

According to another feature of the invention, the device is provided with a gripping means comprising two tongues longitudinally made on the walls of the distal end of the inner tube, elongated in both concave portions and configured in such a way that, being said gripping means in a opening position, when controls are manipulated, the outer tube moves longitudinally in relation to the inner tube in the distal direction, in such a way that the edge of the outer tube press the tongues on the their outer face and, consequently, the concave portions are brought closer together and fitted together, until getting a closing position.

Another significant feature of the medical device object of the invention is that, in the closing position of the gripping means, the outer tube cases fully the inner tube tongues protruding from the edge of its distal end in such a way that, once the sample has been provided inside the concave portions, said edge abuts against the wall of mucus in the area of extraction of the tissues and pushes it away from the sample thus assisting its tear.

Thanks to the combination of the above features, it is possible to take samples of a larger size, and more easily, than those taken by the state-of-art devices.

According to another feature of the invention the medical device is made up of disposable material. In fact, due to the above indicated features, an embodiment of the medical device can be carried out considerably simplified in comparison with those from the state of art, and the manufacturing costs for its disposable embodiment are minimal. In this way it can be guaranteed that the medical device will be in optimal condition at the time of use in terms of hygiene and functioning.

According to another feature of the invention all the composing parts are made up of plastic.

In accordance with another feature of the invention the closed container is removably.

According to another feature of the invention the closed container comprises a screw thread suitable to receive a lid by thread engagement.

### Brief description of the drawings

The attached diagrams illustrate, as a non-limiting example, a preferred embodiment mode of the medical device object of the invention. In said drawings:
Fig. 1 is a schematic view of the medical device;
Fig. 2 is a detailed cross-section view of the gripping means in opening position;
Figs. 3 and 4 are views equivalent to that in Fig. 2, being the gripping means half-opened;
Fig. 6 is a view equivalent to those in Figs. 2 to 5 with the gripping means in a closed position; and
Fig. 7 is a detailed cross-section view of the container of the medical device when a sample is deposited inside.

### Detailed description of the drawings

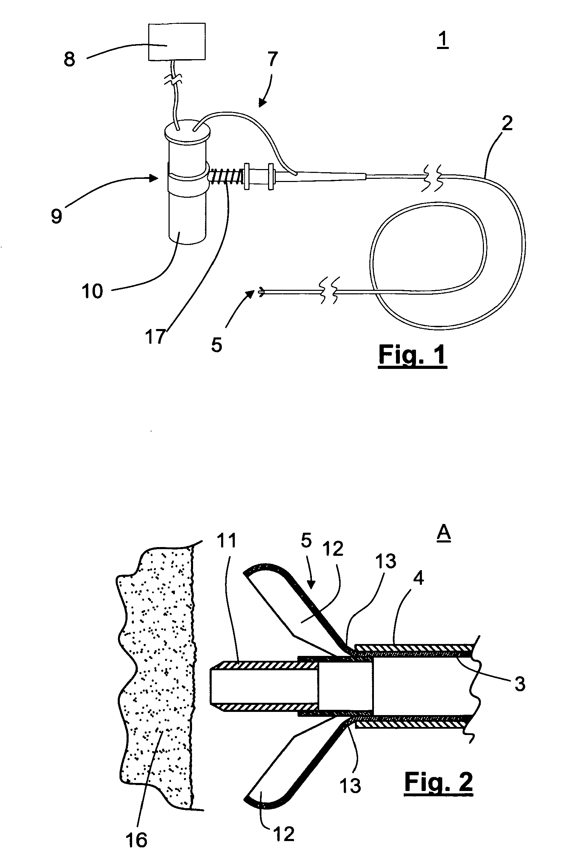

In Fig. 1 can be seen a medical device 1 for biopsies provided with an elongated flexible section 2, a gripping means 5 and some controls 7. Said elongated flexible section 2 is designed to be inserted through a conduit of a surgical instrument, such as for example an endoscope, a laparoscope, etc., and comprises an inner tube 3 and an outer tube 4 which wrap it and is capable of moving longitudinally in relation to the inner tube 3. The gripping means 5, which is located at the distal end of the elongated flexible section 2, is designed to be activated by the controls 7 in order to take a sample, as shown in Figs. 2 to 7.

By the handling of the controls 7, located at the proximal end of the elongated flexible section 2, the gripping means 5 is activated so that it adopts an opening position A or closing position B. Said controls 7 comprise a handle 9 provided with a closed container 10 to which are connected a suction means 8 and in a sealed form the proximal end of the inner tube 3 of the elongated flexible section 2.

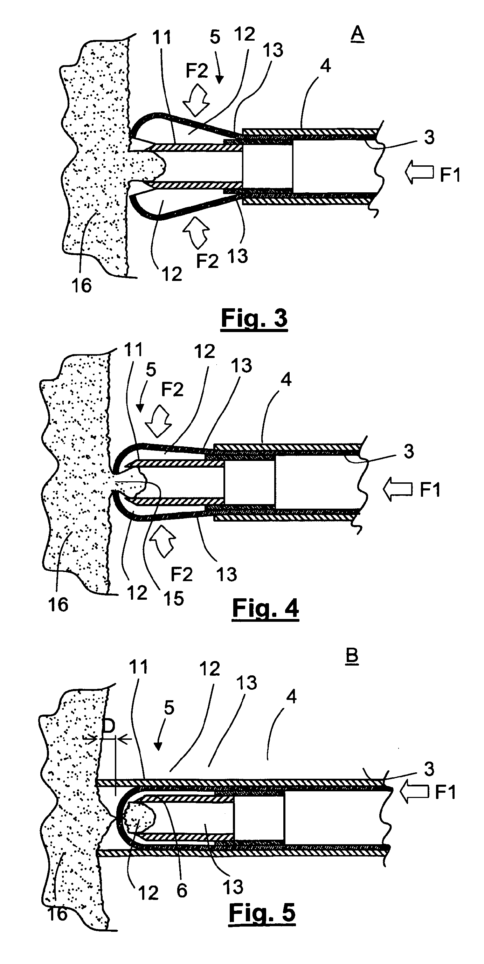

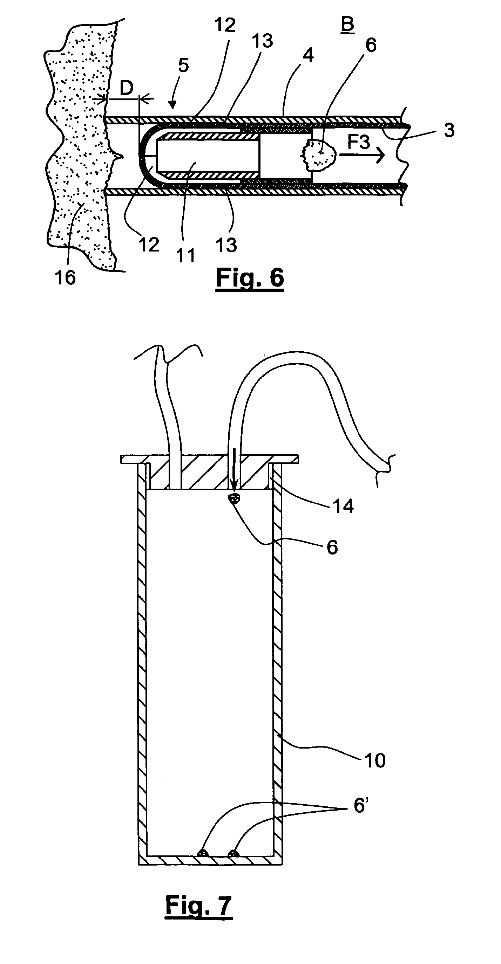

The controls 7 are preferably provided with a spring 17, placed coaxially with the inner and outer tubes 3 and 4 respectively, which keeps the distal end of the outer tube 4 protruding above the distal end of the inner tube 3, with the gripping means 5 remaining concealed and closed, as can be seen in Figs. 5 and 6. When the spring 17 is compressed, the outer tube 4 moves in relation to the inner tube 3 in such a way that the gripping means 5 is shown and opened by elastic reaction, as has been illustrated in Fig. 1. When the compression ceases, the spring 17 recovers its initial form and again places the outer tube 4 as it was initially, totally concealing the gripping means 5.

Below is described the method used by the medical device 1 when taking a sample 6 of tissue 16, once the medical device 1 has already been introduced into the conduit of a surgical instrument not shown, and having reached the area from which it is intended to extract the tissue, special attention being given to the various different positions adopted by the gripping means 5 of the medical device 1.

In Fig. 2 the gripping means 5 is shown just before taking a sample 6, being the gripping means 5 in a opened position A. The gripping means 5 is provided with a cannula 11 arranged at the distal end of inner tube 3 and comprises two tongues 13, made on the walls of the distal end of inner tube 3, elongated in concave portions 12. In a variant of the invention, the distal end of the inner tube 3 is provided with a configuration that, in the closed position, a substantially spherical head wherein a cut is made according to a diametrical plane which divides it into two halves, separable by the section plane, which are initially spaced apart in the opening position 12 and are closed in the operational process of a biopsy, being determined in the end of the inner tube 3 said concave portions 12.

As can be seen in Fig. 2, in opened position A the distal ends of the concave portions 12 are spaced to each other, exposing the cannula 11, the position which they tend to adopt when no force is being applied to them, due to their partially or totally elastic conformation.

When the gripping means 5 comes closer and contact with the tissue 16, the force of suction generated by the suction means 8 produces a depression in the mouthpiece of the cannula 11, thus attracting a portion of mucus from the tissue 16 while at the same time positioning firmly in place the gripping means 5 on the surface of the tissue 16, as shown in Fig. 3. At the same time the controls 7 are activated in such a way that the outer tube 4 moves longitudinally in relation to inner tube 3 in the direction indicated by the arrow F1. Consequently the edge of the distal end of outer tube 4 press the tongues 13 on their outer face, for which reason the concave portions 12 start to come closer together, as indicated by the arrows F2.

While the outer tube 4 keeps moving in the manner described, as indicated by arrow F1, the concave portions 12 keeps moving closer and closer together, as indicated by arrows F2. There comes a point at which the concave portions 12 grip the neck 15 of the section of mucus, acting as a pincer, since due to the force of suction, the portion of mucus protrudes slightly by way of protuberance towards the mouthpiece of the cannula 11 of the gripping means 5 (see Fig. 4). Although this is not shown, the possibility has been considered that the edges of the concave portions 12 comprise an indented finish, so that the teeth can fasten onto the neck 15 of the mucus.

Meanwhile, the outer tube 4 keeps advancing in relation to inner tube 3, as indicated by arrow F1, so that the concave portions 12 of the gripping means 5 continue to come closer together, until outer tube 4 completely conceals gripping means 5 (as observed in Fig. 5). In this position, the edge of the distal end of the outer tube 4 press the tongues 13 on their outer face, so that the concave portions 12 touch one another and fit together, said edge keeping the tongues pressed in closing position B, in the manner of closed jaws.

Unlike other known devices of its kind, the concave portions 12 have not been designed to perform a cut in the tissue 16. For this reason the material of which they are made up does not need to have a very high degree of hardness, and may be of the same material as the inner tube 3; nor does it require an especially sharp finish. As a result of this simplification, at the moment shown in Fig. 5 at which the sample 6 is gripped, the latter may still be joined to the tissue 16 by fibrous threads that are difficult to break, in spite of the pressure exercised by the concave portions 12 of the gripping means 5.

In order to solve the problem indicated above, in the closing position B of the gripping means 5 (see Fig. 5), the outer tube 4 keeps moving in relation to the inner tube 3 as indicated by arrow F1, and is extended until it totally conceals the gripping means 5, protruding from the edge of its distal end considerably in such a way that the edge abuts against the wall of tissue 16 and keeps it away from said sample 6. Simultaneously, the suction continues, although due to the fact that the end is closed or obstructed, a greater depression is produced inside which contributes to stretch sample 6 towards inside of the gripping means 5.

Since the outer tube 4 keeps moving in accordance with arrow F1, the distance D between the distal end of the concave portions 12 and the surface of the tissue 16 increases, in such a way that sample 6 finishes by being fully torn away from the tissue 16, as it is shown in Fig. 6. It is also observed that the sample 6 taken, by now completely torn away from tissue 16, is pulled through the inner tube 3 by the force of suction generated, as indicated by arrow F3.

In Fig. 7 it can be observed how, when the sample 6 reaches the end of the inner tube 3, it is deposited directly in the container 10. At this moment another sample 6 can be taken again by repeating the previous steps illustrated between Figs. 2 and 7. When the number of samples 6 required has already been taken, the container 10 is removed from the handle 9 of the medical device 1, and can be used as a container for transporting the samples taken 6 for subsequent analysis. Optionally, the container comprises a screw thread 14 designed to receive a lid once separated from the handle 9 of the medical device 1.

Once the use of the medical device described has come to an end, the possibility is intended that the device may be disposed of, for which reason the materials used in all or part of its component parts are disposable, being made, for example, up of plastic.

It has been observed that the device object of the invention may be used for all kinds of endoscopies, especially for the performing of heart biopsies, laparoscopies or hysteroscopies, as well as for viewing, sample taking and drainage, with certain measures being adopted in each case which do not, however, modify the essence of the invention.

## Claims

1. A medical device (1) for biopsies, comprising an elongated flexible section (2) designed to be inserted through a conduit through a conduit of a surgical instrument, comprising said elongated section an inner tube (3) and outer tube (4) each capable of moving longitudinally with respect to the other; a gripping means (5) for taking a biological sample (6), located at the distal end of the elongated section and designed to adopt a first opening position (A) and an operating closing position (B); some controls (7) located at the proximal end of the elongated section, for manipulating the medical device; and a suction means (8) suitable to suck the sample (6) and pull it through the inner tube (3), **characterized in that** the controls comprise a handle (9) provided with a closed container (10) to which are connected in a sealed form the proximal end of the inner tube of the elongated flexible section and the suction means, in such a way that the sample pulled through the inner tube it is deposited in said container.

2. A medical device (1) for biopsies according to claim 1, **characterized in that** the gripping means (5) are provided with a cannula (11) located at the distal end of the inner tube (3) through which the sample (6) is sucked.

3. A medical device (1) for biopsies according to claims 1 or 2, **characterized in that** the gripping means (5) formed integrally with the inner tube (3), being formed by two tongues (13), longitudinally made on the walls of the distal end of the inner tube (3), elongated in both concave portions (12) configured in such a way that, being said gripping means in the opening position (A), when the controls (7) are manipulated the outer tube (4) moves longitudinally in relation to the inner tube in the distal end direction, in such a way that the edge of the outer tube press said tongues on the their outer face and, consequently, the concave portions are brought closer and fitted together, in the manner of jaws, in the closing position (B).

4. A medical device (1) for biopsies according to claim 3, **characterized in that** in the closing position (B) of the gripping means (5), the outer tube (4) cases fully the gripping means protruding from the edge of its distal end in such a way that, once the sample (6) has been provided inside the gripping means (5), said edge abuts against the wall of mucus in the area of extraction of the tissues and pushes it away, moving away the tongues from said sample and assisting the tissues tear.

5. A medical device (1) for biopsies according to any of the preceding claims, **characterized in that** it is made up of disposable material.

6. A medical device (1) for biopsies according to any of the preceding claims, **characterized in that** the parts of which it is made up are of plastic material.

7. A medical device (1) for biopsies according to any of the preceding claims, **characterized in that** the closed container (10) is removably.

8. A medical device (1) for biopsies according to claim 7, **characterized in that** the closed container (10) comprises a screw thread (14) suitable to receive a lid by thread engagement.
